# EUROPEAN PATENT SPECIFICATION

(11) **EP 4 539 811 B1**
(45) Date of publication and mention of the grant of the patent: **25.03.2026**
(21) Application number: 23734751.3
(22) Date of filing: 13.06.2023
(51) Int. Cl.: A61K 8/04, A61K 8/26, A61K 8/25, A61K 8/22, A61Q 11/02

(54) **HYDROGEN PEROXIDE TEETH WHITENING GEL**
WASSERSTOFFPEROXID-ZAHNAUFHELLUNGS-GEL
GEL DE BLANCHIMENT DENTAIRE AU PEROXYDE D'HYDROGÈNE

(30) Priority: 15.06.2022 IT 202200012626
(43) Date of publication of application: 23.04.2025
(73) Proprietor: IDS RESEARCH SOCIETÀ A RESPONSABILITÀ LIMITATA, 16122 Genova (IT)
(72) Inventor: PIERGALLINI, Remigio, 63066 GROTTAMMARE (AP) (IT); LOUPIS, Nikolaos, 14562 KIFISIA (GR)
(74) Representative: Cutropia, Gianluigi
(86) International application number: PCT/IB2023/056075
(87) International publication number: WO 2023/242724

(56) References cited:
- WO-A2-2007/123729
- KR-A- 20150 057 718
- US-A1- 2004 241 110

## Description

The present invention relates to a hydrogen peroxide dental whitening gel.

Peroxides are generally used in the form of gels for the whitening of human teeth. They can be applied at home with the use of custom-made night masks or in the dental surgery where they are applied directly onto the tooth surface by the dental surgery staff.

Peroxides of different compositions gradually decompose, releasing molecular oxygen and active forms of oxygen. The active forms normally undergo a spontaneous degradation, but they are not abundant due to the action of the antioxidant enzymes in the saliva and the gingival crevicular fluid.

The ingredients of a whitening gel used in the dental surgery must be more aggressive in order to release more active forms of oxygen in less time and immediately achieve an acceptable whitening result in one dental session of average duration. In order to accomplish such a purpose, the ingredients must be generally activated by means of physical or chemical activators or catalysts.

The most commonly used chemical activators are various metal ions, such as iron ions or the like, such as alkaline solutions mixed with peroxide gel. Peroxides decompose more rapidly in an alkaline environment, generating oxygen, even though the presence of radical forms of oxygen is limited.

With reference to the prior art, it has been noted that the active forms of oxygen are much more effective for dental whitening. The more radicals are produced, the better the whitening effect will be.

Photoabsorbent molecules, such as biologically acceptable dyes (CRAS: commonly recognized as safe), are used to intensify the dental whitening effect and to amplify active oxygen production.

However, the hydrogen peroxide gels of the prior art are impaired by some drawbacks which are mainly due to the almost immediate release of hydrogen peroxide (H2O2) in liquid form. This is caused by the oxidation of thickeners (carbopolymers, glycerol and propylene glycol) contained in the gel, which is especially accelerated by the mouth temperature (37°C) that is generally higher than the room temperature.

In addition, the hydrogen peroxide in liquid phase flows freely on the teeth, reaches the gingivae and penetrates into the gingival grooves, causing gingival irritation and burning. In an attempt to solve such a problem, gingival protective compounds are used, which are commonly defined as "gingival barriers", although they do not completely solve the problem.

WO2007/123729 discloses a dental whitening gel according to the preamble of independent claim 1.

The purpose of the present invention is to eliminate the drawbacks of the prior art by providing a hydrogen peroxide dental whitening gel that is capable of increasing the whitening efficacy of the hydrogen peroxide contained in the gel.

Another purpose is to provide such a hydrogen peroxide dental whitening gel that is able to ensure a good dispersion and stabilization of the hydrogen peroxide in the gel, avoiding the immediate release of hydrogen peroxide in liquid form when the gel comes in contact with the teeth.

These purposes are achieved in accordance with the invention with the features of the appended independent claim 1.

Advantageous achievements of the invention appear from the dependent claims.

Further features of the invention will appear clearer from the following detailed description, which refers to a merely illustrative and therefore nonlimiting embodiment, illustrated in the following examples.

The whitening gel according to the invention comprises:
A) a hydrogen peroxide aqueous solution,
B) colloidal silicon dioxide in amorphous form,
C) potassium and aluminum silicate, and
D) crystalline aluminum dioxide.

The hydrogen peroxide aqueous solution comprises hydrogen peroxide (H2O2) in a weight percentage comprised between 10% and 50% relative to the total weight of the aqueous solution.

Colloidal silicon dioxide is also known as hydrophilic pyrogenic silica because it is obtained by hydrolysis in gaseous phase of silicon tetrachloride (SiCl4). The colloidal silicon dioxide absorbs and retains water. The colloidal silicon dioxide is in the amorphous form (random distribution of atoms). This means that, unlike crystalline silica, it is not dangerous in case of accidental inhalation. The colloidal silicon dioxide is in the form of micronized powder with a particle size of 0.2-0.3 µm.

The whitening gel comprises colloidal silicon dioxide in a weight percentage comprised between 0.1% and 7%, preferably 2%-5%, relative to the total weight of the gel.

The potassium and aluminum silicate preferably comprises mica powder in crystalline form. Mica crystals are generally tabular with diamond or hexagonal section with angles of approximately 60° and 120°, and assume pseudo-orthorhombic or pseudo-hexagonal morphologies.

Potassium and aluminum silicate can also be a food additive, known as E555, used as a carrier of E171 (titanium dioxide) and E172 (iron oxides and hydroxides) dyes.

The whitening gel comprises potassium and aluminum silicate in a weight percentage comprised between 0.1% and 5%, preferably 1%-3%, relative to the total weight of the gel.

Aluminum dioxide (Al203) in crystalline form is preferably corundum powder. Corundum has a crystalline form with tabular, or prismatic or barrel habitus (by bipyramid sequence).

The whitening gel comprises aluminum dioxide in a weight percentage comprised between 0.1% and 5%, preferably 1%-3%, relative to the total weight of the gel.

In order to obtain the whitening gel, firstly the solid components (colloidal silicon dioxide, potassium and aluminum silicate, and aluminum dioxide) are mixed.

Then, the mixture of solid components is poured into the hydrogen peroxide aqueous solution, and a planetary mixing with a mixer is performed. In this way a product in the form of a gel is obtained.

With the addition of the hydrogen peroxide aqueous solution, the crystalline molecules contained in the mixture of solid components produce a gel, in which the hydrogen peroxide aqueous solution is attracted by the crystalline molecules so as to surround said crystalline molecules, forming an atmosphere that surrounds the crystalline molecules. Light can penetrate said hydrogen peroxide atmosphere, increasing the decomposition of the hydrogen peroxide into water and oxygen, via the transition of the oxygen to reactive oxygen species (ROS).

The whitening gel is applied directly onto the teeth to be whitened, using special application devices, such as syringes and tips.

Preferably, the whitening gel according to the invention can be used together with a photo-activating composition that contains photo-reactive molecules, for the purpose of increasing the whitening action of the oxygen contained in the hydrogen peroxide of the whitening gel.

The whitening gel can be applied onto the surface of the teeth to be whitened alone. However, in such a case, it will lack the acceleration of the whitening process offered by the photo-activation. Otherwise said, the whitening gel will whiten the teeth in a longer time than when used together with a photo-activating composition.

In any case, when the whitening gel is applied alone, the decomposition of the hydrogen peroxide (H2O2) in water (H2O) and oxygen (02) occurs gradually, releasing reactive oxygen species (ROS).

The reactive oxygen species (ROS) oxidize the organic residues (dyschromic compounds) that have penetrated into the dental enamel and the teeth will gradually appear whiter.

The whitening gel comprises inert crystalline materials (colloidal silicon dioxide, potassium and aluminum silicate, and aluminum dioxide). It should be noted that the silicon dioxide crystals are amorphous crystals, that is, they do not follow a repeatable crystal structure with the same stereometric shape, and the material is inert.

As a result, electrostatic forces are generated around the crystalline molecules of such crystalline materials, which tend to retain the liquid part of the hydrogen peroxide for a long time. That is, liquefaction of the gel does not occur.

Such a phenomenon maintains and prolongs the presence of the active oxygen in its reactive oxygen species (ROS), with the presence of a highly reactive environment that causes a prolonged oxidation of the organic residues that have penetrated into the dental enamel. This leads to a surprisingly higher whitening power of the gel according to the invention compared to the whitening gels of the prior art.

Another advantage of the whitening gel according to the invention is that the hydrogen peroxide does not flow freely on the teeth in liquid phase. This prevents gingival irritation and burning. Thus, the whitening gel is both more effective in terms of whitening effect and safer (it does not cause gingival irritation).

The whitening gel according to the invention makes it possible to drastically solve a major clinical problem, namely the leaking of liquid peroxide that penetrates the gingival grooves of the teeth, and requires the use of protective compounds (gingival barriers).

## Claims

1. Dental whitening gel comprising:
A) a hydrogen peroxide aqueous solution, and
B) colloidal silicon dioxide in amorphous form,
**characterized in that**
said dental whitening gel further comprises:
C) potassium and aluminum silicate, also known as potassium aluminium silicate, and
D) aluminum dioxide, chemical formula Al2O3, in crystalline form.

2. The whitening gel according to claim 1, wherein said gel is made with a hydrogen peroxide aqueous solution comprises hydrogen peroxide (H2O2) in a weight percentage comprised between 10% and 50% relative to the total weight of the aqueous solution.

3. The whitening gel according to claim 1 or 2, wherein the colloidal silicon dioxide, the potassium and aluminum silicate, and the aluminum dioxide, chemical formula Al2O3, are in powder form.

4. The whitening gel according to claim 3, wherein the colloidal silicon dioxide is in the form of micronized powder with a particle size of 0.2-0.3 µm.

5. The whitening gel according to any one of the preceding claims, comprising colloidal silicon dioxide in a weight percentage comprised between 0.1% and 7%, preferably 2%-5%, relative to the total weight of the gel.

6. The whitening gel according to any one of the preceding claims, wherein said potassium and aluminum silicate comprises mica powder.

7. The whitening gel according to any one of the preceding claims, wherein said potassium and aluminum silicate is a food additive, known as E555.

8. The whitening gel according to any one of the preceding claims, comprising potassium and aluminum silicate in a weight percentage comprised between 0.1% and 5%, preferably 1%-3%, relative to the total weight of the gel.

9. The whitening gel according to any one of the preceding claims, wherein said aluminum dioxide, chemical formula Al2O3, is corundum powder.

10. The whitening gel according to any one of the preceding claims, comprising aluminum dioxide, chemical formula Al2O3, in a weight percentage comprised between 0.1% and 5%, preferably 1%-3%, relative to the total weight of the gel.

## Patentansprüche

1. Zahnaufhellungsgel, umfassend:
A) eine wässrige Lösung von Wasserstoffperoxid und
B) kolloidales Siliciumdioxid in amorpher Form,
**dadurch gekennzeichnet, dass**
das Zahnaufhellungsgel ferner umfasst:
C) Kalium- und Aluminiumsilikat, auch bekannt als Kaliumaluminiumsilikat,
D) Aluminiumdioxid, chemische Formel Al₂O₃, in kristalliner Form.

2. Zahnaufhellungsgel nach Anspruch 1, wobei das Gel mit wässriger Lösung von Wasserstoffperoxid hergestellt ist, Wasserstoffperoxid (H₂O₂) in einem Gewichtsprozentsatz im Bereich von 10 bis 50 %, bezogen auf das Gesamtgewicht der wässrigen Lösung umfasst.

3. Zahnaufhellungsgel nach Anspruch 1 oder 2, wobei das kolloidale Siliciumdioxid, das Kaliumaluminiumsilikat und das Aluminiumdioxid, chemische Formel Al₂O₃, in Pulverform vorliegen.

4. Zahnaufhellungsgel nach Anspruch 3, wobei das kolloidale Siliciumdioxid ein mikronisiertes Pulver mit einer Korngröße von 0,2 bis 0,3 µm ist.

5. Zahnaufhellungsgel nach einem der vorstehenden Ansprüche, umfassend kolloidales Siliciumdioxid in einem Gewichtsprozentsatz im Bereich von 0,1 bis 7 %, vorzugsweise 2 bis 5 %, bezogen auf das Gesamtgewicht des Gels.

6. Zahnaufhellungsgel nach einem der vorstehenden Ansprüche, wobei das Kaliumaluminiumsilikat Glimmerpulver umfasst.

7. Zahnaufhellungsgel nach einem der vorstehenden Ansprüche, wobei das Kaliumaluminiumsilikat ein Lebensmittelzusatzstoff ist, der als E555 bekannt ist.

8. Zahnaufhellungsgel nach einem der vorstehenden Ansprüche, umfassend Kaliumaluminiumsilikat in einem Gewichtsprozentsatz im Bereich von 0,1 bis 5 %, vorzugsweise 1 bis 3 %, bezogen auf das Gesamtgewicht des Gels.

9. Zahnaufhellungsgel nach einem der vorstehenden Ansprüche, wobei das Aluminiumdioxid, chemische Formel Al₂O₃, Korundpulver ist.

10. Zahnaufhellungsgel nach einem der vorstehenden Ansprüche, umfassend Aluminiumdioxid, chemische Formel Al₂O₃, in einem Gewichtsprozentsatz im Bereich von 0,1 bis 5 %, vorzugsweise 1 bis 3 %, bezogen auf das Gesamtgewicht des Gels.

## Revendications

1. Gel de blanchiment dentaire comprenant :
A) une solution aqueuse de peroxyde d'hydrogène, et
B) du dioxyde de silicium colloïdal sous forme amorphe,
**caractérisé en ce que**
ledit gel de blanchiment dentaire comprend en outre :
C) du silicate d'aluminium et de potassium, aussi connu sous le nom de silicate aluminopotassique,
D) du dioxyde d'aluminium, formule chimique Al₂O₃, sous forme cristalline.

2. Gel blanchissant selon la revendication 1, où ledit gel est composé d'une solution aqueuse de peroxyde d'hydrogène et comprend du peroxyde d'hydrogène (H2O2) avec un pourcentage poids compris entre 10% et 50% par rapport au poids total de la solution aqueuse.

3. Gel blanchissant selon la revendication 1 ou 2, où le dioxyde de silicium colloïdal, le silicate d'aluminium et de potassium et le dioxyde d'aluminium, formule chimique Al₂O₃, sont présents sous forme de poudre.

4. Gel blanchissant selon la revendication 3, où le dioxyde de silicium colloïdal est présent sous forme de poudre micronisée avec une granulométrie comprise entre 0,2-0,3 µm.

5. Gel blanchissant selon l'une quelconque des revendications précédentes, comprenant du dioxyde de silicium colloïdal ayant un pourcentage poids compris entre 0,1% et 7%, préférablement 2%-5% par rapport au poids total du gel.

6. Gel blanchissant selon l'une quelconque des revendications précédentes, où ledit silicate d'aluminium et de potassium comprend de la poudre de mica.

7. Gel blanchissant selon l'une quelconque des revendications précédentes, où ledit silicate d'aluminium et de potassium est un additif alimentaire, connu sous le sigle E555.

8. Gel blanchissant selon l'une quelconque des revendications précédentes, comprenant du silicate d'aluminium et de potassium ayant un pourcentage poids compris entre 0,1% et 5%, préférablement 1%-3% par rapport au poids total du gel.

9. Gel blanchissant selon l'une quelconque des revendications précédentes, où ledit dioxyde d'aluminium, formule chimique Al₂O₃, est de la poudre de corindon.

10. Gel blanchissant selon l'une quelconque des revendications précédentes, comprenant du dioxyde d'aluminium, formule chimique Al₂O₃, ayant un pourcentage poids compris entre 0,1% et 5%, préférablement 1%-3% par rapport au poids total du gel.
